Europäisches Patentamt

(19) European Patent Office

Office européen des brevets

(11) Publication number: **0 057 793**
**B1**

(12) **EUROPEAN PATENT SPECIFICATION**

(45) Date of publication of patent specification: **23.04.86**

(51) Int. Cl.⁴: **A 61 F 2/30**

(21) Application number: **81305878.1**

(22) Date of filing: **14.12.81**

(54) Joint prosthesis, particularly an elbow prosthesis.

(30) Priority: **12.12.80 GB 8039835**

(43) Date of publication of application:
**18.08.82 Bulletin 82/33**

(45) Publication of the grant of the patent:
**23.04.86 Bulletin 86/17**

(84) Designated Contracting States:
**CH DE FR GB LI**

(56) References cited:
**EP-A-0 006 314
DE-A-2 529 238
GB-A-1 528 906
US-A-3 708 805
US-A-3 852 831
US-A-3 919 725
US-A-4 057 858
US-A-4 079 469**

(73) Proprietor: **Wadsworth, Thomas Gordon
22 Hyde Park Square
London, W2 2NL (GB)**

(72) Inventor: **Wadsworth, Thomas Gordon
22 Hyde Park Square
London, W2 2NL (GB)**

(74) Representative: **Burrows, Anthony Gregory
et al
Haseltine Lake & Co. Hazlitt House 28
Southampton Buildings Chancery Lane
London, WC2 1AT (GB)**

## Description

This invention relates to prostheses.

In general, prosthetic replacement of the elbow has proved a difficult and often disappointing task over the years and this has been the experience of most surgeons in this field. In the past, it was common to replace the elbow joint by means of a constrained prosthesis, usually consisting of a large stem inserted into the humerus and another into the ulna and the two parts of the component linked together by an axle pin. The strong forces on the elbow tended to disrupt the prosthesis from the bone and a very great deal of bone had to be removed to put in such a prosthesis, resulting in the long-term in a disastrous situation very often if the prosthesis has, in fact, to be removed.

There has been a move in recent times to surface replacement of the elbow joint and my work in this area led to the development of devices which are described in U.S. patent 4,079,469 and European Patent Application Publication 0006314. Numerous other patents have issued describing bone joint prostheses of which the following is a representative listing:

| U.S. 2,784,416 | U.S. 3,840,905 |
| --- | --- |
| 3,547,115 | 3,582,831 |
| 3,748,662 | 3,869,729 |
| 3,798,679 | 3,886,599 |
| 3,801,990 | 3,919,725 |
| 3,816,854 | 3,990,116 |
| | 4,038,704 |
| | 4,057,858 |
| U.K. 1444724 | |

Various ways are known of fixing a plastics prosthetic articular head to a metal prosthetic stem. For example, United States Patent 3,816,854 discloses a humeral component consisting of a generally cylindrical head of ultra high density polyethylene and a chrome-nickel-cobalt alloy stem incorporating at its distal end a hollow partial cylinder which closely embraces the head. Shallow protuberances on the inner surface of the partial cylinder engage in shallow depressions in the outer surface of the head to retain the head on the stem. Unfortunately, the reliability of the retention of the head in the partial cylinder is relatively low, depending as it does upon the protuberances and the depressions. United States Patent 4,131,956 describes a humeral component consisting of a generally cylindrical internally threaded head of a biocompatible plastics, a forked stem of biocompatible metal, and two screws screwed into the threaded interior of the head through the respective limbs of the fork of the stem. Such a forked stem has the disadvantage of requiring removal of a considerable amount of bone.

In order to facilitate implanting of an elbow prosthesis, it is known from United States Patent 3708805 to make the ulnar component in two separate parts, namely a stem and a head, fastenable together. The stem has at its proximal end a tenon portion formed with a transverse bore. The head has at its proximal end a hinge part for receiving a hinge pin and at its distal end two prongs which embrace the tenon portion and are formed with respective bores which are countersunk at their outer ends and which can be brought into alignment with the bore in the tenon portion. A rivet inserted through the bores fastens the head to the stem. Flaring-out of the rivet is done by a surgeon applying a suitably designed G-clamp across the ends of the rivet. The fit of the tenon portion in the head is of an accuracy such as to provide a substantially rigid fit between the head and the stem.

However, the reliability of the fastening depends upon the flaring-out of the rivet. If, because of incorrect positioning of the rivet which is easily done, the G-clamp fails to flare-out the rivet sufficiently, then the rivet can work out laterally or medially, with serious results for the patient.

According to the present invention, there is provided a prosthetic component comprising an articular head, a first hole of closed cross-section extending in said head from a first surface zone of said head, a second hole extending in said head from a second surface zone of said head and joining said first hole, a stem extending along said second hole and formed with an aperture therethrough aligned with said first hole, and a member extending in said first hole and said aperture and retaining said head on said stem, characterized in that said first hole is closed at its end remote from said first surface zone and is closed at said first surface zone by a plug therein.

Thereby, not only can the head be reliably retained on the stem, but the amount of bone needing to be removed can be minimal.

In order that the invention may be clearly understood and readily carried into effect, reference will now be made by way of example to the accompanying drawings, in which:—

Figure 1 is a fragmentary anterior elevation of a known elbow prosthesis illustrated as implanted in the humerous and ulna.

Figure 2 is a fragmentary lateral elevation of the elbow prosthesis with humerus and ulna in extension position (0°).

Figure 3 is a perspective view of an ulnar component of the prosthesis.

Figure 4 is a perspective view of a humeral component of the prosthesis.

Figure 5 is a perspective view of a humeral component impactor.

Figure 6 is a perspective view of a known ulnar component impactor.

Figure 7 is a perspective view of a known chisel used to cut the humerus for implanting the humeral component.

Figure 8 is a perspective view of one known embodiment of a trial humeral component used primarily for replacement of a totally destroyed elbow joint.

Figure 9 is a perspective view of another em-

bodiment of a trial humeral component.

Figure 10 is a perspective view of another known chisel used in implanting the humeral component.

Figure 11 is a plan view of the known usual form of humeral component of the prosthesis.

Figure 12 is a plan view of the known special form of humeral component used for replacement of a totally destroyed elbow joint.

Figure 13 is a fragmentary perspective view of the chisel cutting the humerus for implantation of the humeral component.

Figure 14 is a fragmentary perspective view of the humeral impactor pushing the humeral component onto the humeral bone during cementing.

Figure 15 is a fragmentary perspective view of the ulnar impactor pushing the ulnar component onto the ulnar bone during cementing.

Figure 16 is a fragmentary perspective view of the trial humeral component of Figure 8 being applied to the humerus.

Figure 17 is a perspective view of a known radial head component that may be used on occasion in the new elbow replacement prosthesis.

Figure 18 is a sectional view taken on the line A—A of Figure 1.

Figure 19 is an elevation in the lateral direction of a modified version of the humeral component constituting an example of the present invention.

Figure 20 is an elevation in the distal direction of the modified version.

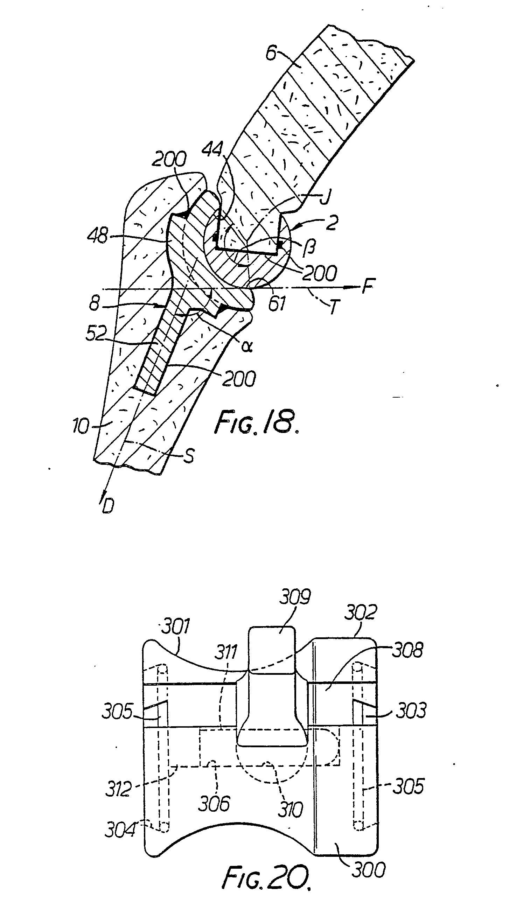

Referring in detail to the drawings, the known elbow replacement prosthesis 2 basically comprises a humeral component 4 that is implanted in the humerus 6 and an ulnar component 8 implanted in the ulna 10. Optionally, it may comprise a radial component 12 (Figure 17) that is implanted in the radius 14.

The humeral component 4 has a surface 16 which is concave in the coronal plane and convex in the sagittal plane and which at its lateral end merges into a cylindrical surface 18, the latter partially replacing the capitulum bone of the humerus upon implantation. At its medial end the surface 16 merges into a convex surface 20. The surfaces 16, 18 and 20 form the articular surfaces of the humeral component 4 relative to the ulnar component and the radial component.

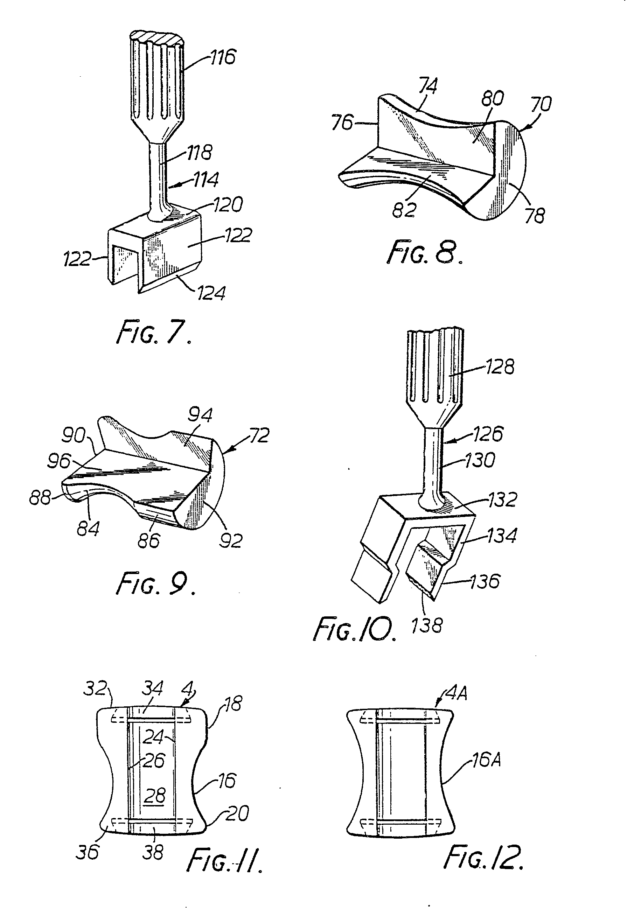

A slightly different form of humeral component 4A is used in the prosthesis for replacement of a totally destroyed elbow joint (see Figure 12). The component 4A is the same in all particulars to component 4 except that the articular surface 16A concave in the coronal plane extends right across the whole length of the component 4A and there is no cylindrical surface such as surface 18 in component 4.

The surface for attaching the humeral component 4 to the humerous comprises a superior U-slot 22 with chordal anterior wall 24, posterior wall 26 and flat floor 28. Longitudinal grooves 30 are formed in the walls 24 and 26 for keying of cement for implantation of the component 4. The lateral wall 32 has a peripheral groove 34 and the medial wall 36 a similar groove 38 for keying of cement. Annular wire markers 40 are imbedded in the walls 32 and 26 and a longitudinal wire marker 42 is embedded in the floor 28 so that position of the prosthesis can be determined by x-ray inspection, after implantation, of both the coronal and sagittal planes by means of the three markers.

The main articulating surface of component 4 describes a gently concave curve in the coronal plane which allows for simple and effective engagement with the convex surface 44 in the coronal plane of the ulnar component 8. Thus, a limited amount of sideway rotation motion is allowed which may at times become necessary in order to avoid undue strain on the bony attachments to the prosthetic components.

In the sagittal plane of the component 4, the convex articulating surface 16 increases in radius from its center in both the medial and lateral directions to a similar extent where the surfaces 18 and 20 take over.

The articular surface 44 of ulnar component 8 allows for accurate and easy articulation with surface 16 of the humeral component 4.

The bone attaching portion of the ulnar component 8 comprises a longitudinal, arcuate keel 48 with grooves 50 running longitudinally along beside the keel 48 for keying of cement in implantation. The keel 48 depends from the undersurface 54 of the arcuate articulating portion of the component 8 and has a dovetailed cross-section in the coronal plane. A stem 52 depends from the keel 48 for insertion into the ulna. The stem 52 has nipples 56 on both the medial surface 58 and lateral surface 60 for keying into cement for implantation.

The orientation of the stem 52 relative to the articular surface of the ulnar component is selected to control anteversion seating of the component 8 in the ulnar bone 10. With reference to Figure 18, the angle $\alpha$ between the forward direction F of the tangent T to the distal end 61 of the articular surface 44 of the ulnar component 8 and the distal direction D of the longitudinal axis S of the stem 52 is more than 90°. Hence the distal end 61 of the articular surface of the ulnar component is inclined forwardly generally in the direction F at an angle of more than 90° to the distal direction D of the stem 52. The advantage of this feature is that forces in the direction 180° to the direction D, which are common in normal use of the joint, do not tend to dislocate the ulnar component posteriorly relative to the humeral component. The risk of such dislocation is an important hazard in elbow replacement surgery and is greatly mitigated by this feature of the prosthesis. Figure 18 also shows that the articular surface of the ulnar component 8 subtends at the axis J of the turning of the joint an angle $\beta$ of less than 180°. The advantage of this feature is that if the joint should become dislocated the two components will not be prevented from returning to a proper position with their respective articular surfaces in contact with one another. Cement between each component and the ulna 10 or the

humerus 6 is indicated in Figure 18 by the solid shading 200.

Making the capitellum surface 18 cylindrical has the advantage that the merging lateral end of the surface 16 provides a high obstruction to lateral displacements of the component 8 relative to the humeral component.

A reconstructed elbow joint may also include a radial component 12 (Figure 17). In actual fact, although the head of the radius 14 is always removed for insertion of the prosthesis, it is not always necessary to replace this removed bone. However, in necessary cases, the bone may be replaced with the radial component 12 which comprises a distal key portion 62 having cement keying grooves 64 and an integral proximal bearing button 66 with dished surface 68. If the component 12 is used, it is implanted into the surgically prepared radius 14 and fixed in place with cement in a manner similar to implant of the ulnar component 8.

In order to assess the depth of humeral bone to be removed and also for sizing of a component to fit a particular patient, trial humeral components may be used, i.e., special component 70 or usual humeral component 72. The component 70 corresponds to the permanent special component 4A and component 72 to the permanent usual component 4.

The component 70 comprises the concave surface 74 in the coronal plane, lateral wall 76 and medial wall 78. Instead of a U-shaped slot as in the permanent component 4A, component 70 has an L-shaped slot defined by the posterior wall 80 and the floor 82.

The component 72 comprises the concave surface 84 in the coronal plane, convex surfaces 86 & 88, medial wall 90, lateral wall 92 and an L-shaped slot defined by posterior wall 94 and floor 96.

The components 70 & 72, unlike their permanent counterparts 4 and 4A do not include wire markers or keying grooves, but, aside from this and the L-shaped slots, components 70 & 72 are the same as 4A & 4.

In reconstruction of an elbow joint, only one style of components 4, 4A or 70 need be used. However, with component 72, right and left hand styles must be used dependent upon whether a right or left elbow is being reconstructed.

A variety of materials are available from which to construct components 4, 8 and 12. Advantageously, the humeral component is formed of inert plastic, e.g. high density polyethylene, and the ulnar component 8 and radial component 12 are made of corrosion-resistant metal, e.g., the chromium alloy "Aluvium". The impactors and chisels are advantageously formed of stainless steel or equivalent metal except for the end of the ulnar impactor in contact with the ulnar prosthesis.

The design of the components 4 and 8 as described enables them to be made and used in an average size adaptable to a large numer of patients. However, it should be recognised that different sizes may be necessary and this can be determined by use of sized trial components 70 or 72.

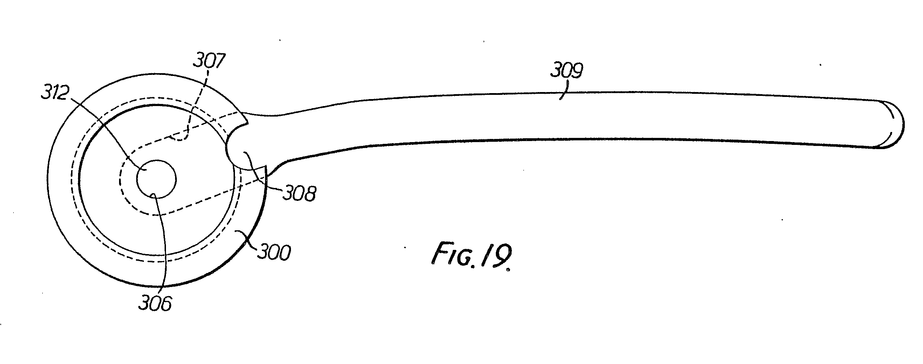

In some cases, it may be desired that the humeral component should comprise a stem for insertion in the humerus. A suitable component is shown in Figures 19 and 20, which is of a lefthand style. It comprises an articular head 300 of inert plastics, with an articular surface 301 similar in shape to the surface 16 and an articular surface 302 similar in shape to the surface 18. In its medial and lateral ends, the head 300 is formed with respective co-axial undercut recesses 303 and 304, the undercut portions of which house wire markers 305. Extending co-axially from the recess 304 is a blind bore 306. This intersects a radial slot 307 which itself intersects a channel 308 forming a discontinuity in the articular surfaces 301 and 302 and serving to engage a suitably cut keel of the humerus in order to improve stability of mounting of the component on the humerus. The component comprises a metal stem 309 of which the distal end fits in the slot 307 and is formed with an aperture 310 aligned with the bore 306. Through the aperture 310 and projecting beyond both ends thereof is a metal pin 311 retaining the stem 309 in the head 300. A plastics plug 312 closes the open end of the bore 306 and is a press-fit therein to prevent the pin 311 from sliding from or within the bore. In assembling the component, the stem 309 is inserted into the slot 307, then the pin 311 is inserted through the aperture 310 and up to the blind end of the bore 306, and then the plug 312 is inserted into the open end of the bore 306 until it is firmly pressed against the pin 311 and is thus flush with the base of the recess 304.

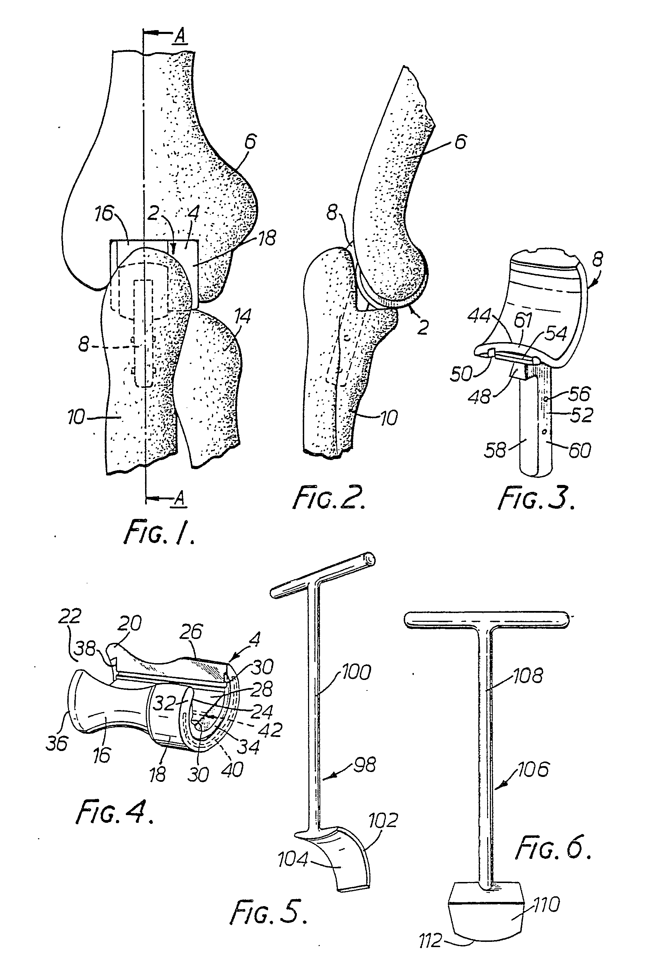

Instrumentation for reconstruction of an elbow joint is shown in Figures 5—7 and 10.

The humeral impactor 98 (or pusher) comprises a T-shaped handle 100 fixed in the coronal plane to the integral distal end member 102 having its pushing surface 104 formed complementarily to the concave-convex surface 16 of component 4 or 300. This enables impactor 98 to be put in position upon the humeral component easily and be held there accurately while cement hardens during implantation.

The ulnar impactor 106 also has a T-shaped handle 108, but this is fixed in the sagittal plane to the integral distal end member 110. The pushing surface 112 is formed complementarily to the articulating surface 44 of component 8 to again provide easy placement and accurate holding during cementing.

Chisel 114 comprises mallet head 116, stem 118, base member 120 and two wings 122, each with chisel tips 124. The stem 118 is perpendicular to the base member 120, but offset from the center thereof for ease of access to the elbow joint. Chisel 114 is used to mark out the antero-posterior breadth of the humeral bone to be removed in order to fit into the slot 22 of component 4. It also marks the depth: equivalent to that of the depth of the slot 22 in component 4.

Chisel 126 comprises mallet head 128, stem 130, base member 132, wings 134 with inset ends 136 and chisel tips 138. The wings 134 are in a different plane from the wings 122 of chisel 114.

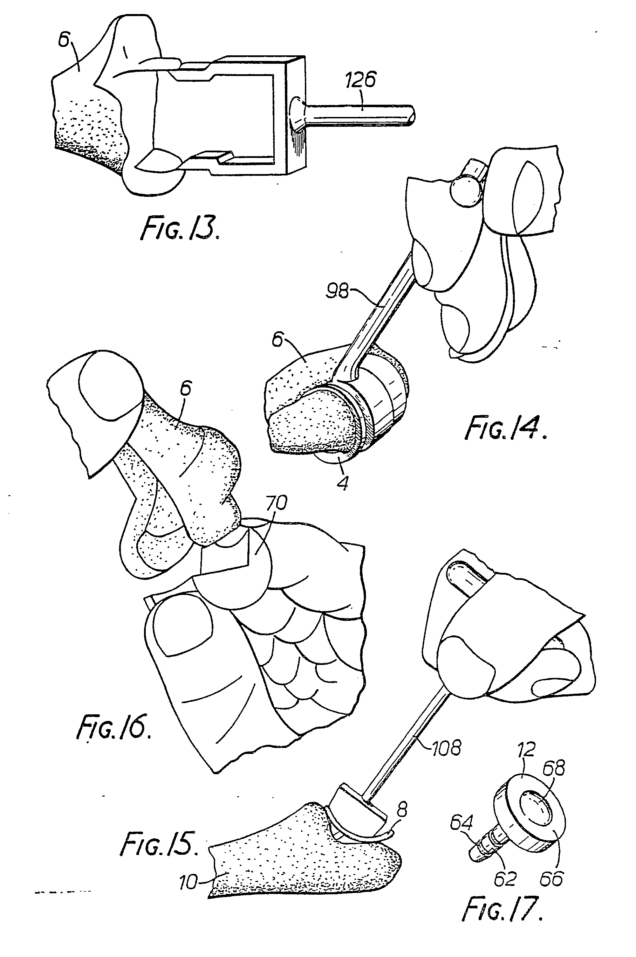

Chisel 126 is used for accurately marking out the length of humeral bone to be removed and also to mark the depth since the inset ends 136 of the chisel are sized in length to compare with the depth of the body of component 4. Use of chisel 126 to mark out and cut away humeral bone from the humerus 6 is shown in Figure 13. Chisel 114 is then used in the opposite plane to mark out the antero-posterior breadth of bone removal. Upon completion of humeral bone removal in such manner, the humeral component 4, with cement applied to the surfaces of slot 22 is applied to the humerus 6 and held in place with impactor 98 as shown in Figure 14 until the cement has set. The keel of the prepared humeral bone sits in the U-slot of component 4 and its medial and lateral ends sit against the medial and lateral walls of the prepared lower humerus.

In implantation of the humeral component 300, the chisel 114 is not employed, but the chisel 126 is used in the same manner as with the component 4.

The ulna is prepared to receive the stem 52 of ulnar component 8. Cement is then applied to the stem 52 and undersurface 54 and the component 8 is inserted into and onto the upper end of the ulna 10. The component 8 is held in position as shown in Figure 15 with impactor 108 until the cement is fully set. In the reconstructed joint, the ulnar component sits on the olecranon process of the ulnar bone and the stem 52 seats within that bone to give good stability to the component 8.

A preferred cement for use in the joint reconstructions is self-hardening methyl methacrylate cement, but other body compatible cements may be used.

As previously indicated, trial humeral components may be used in order to assess the depth of humeral bone to be removed and also for sizing of a component to fit a particular patient. Figure 16 illustrates the application of the trial component 70 to the surgically prepared humeral bone 6.

It is to be understood that Figures 13—16 are illustrative only and do not attempt to depict the actual appearance of the operations. Obviously, the actual surgical procedures would be conducted under sterile conditions with the surgeon' hands gloved and there would flesh around the humerus and ulna.

In the reconstructed elbow joint, the articulating surfaces, e.g. 16 and 44, fit easily together and give a range of flexible movement between full extension (0°) to flexion position (140°). Stability is provided, in particular, by the intact medial ligament of the elbow and also by the lateral ligament, the anterior and posterior joint capsule and the adjacent musculature. Hence, any tendency for dislocation of the prosthesis is resisted by the natural anatomy and any lateral shift of the ulna is discouraged by the lateral cylindrical part 18 or 302 of the humeral component 4 or 300. This is useful if trauma or undue strain is delivered to the elbow joint. Further, the prosthetic articulating arrangement allows for rotation of the ulna at the elbow, thus avoiding strain on the seating of the two components in strain situations.

Minimal bone is removed in the operative procedure which is a distinct advantage if there should be failure of the prosthesis for any reason, in which event, other surgical procedures can be easily accomplished.

The humeral wire markers allow for accurate visualisation of the prosthesis by x-ray which is important in assessing wear and/or displacement after accidental trauma or loosening of the humeral component for any reason.

## Claims

1. A prosthetic component comprising an articular head (300), a first hole (306) of closed cross-section extending in said head from a first surface zone of said head (300), a second hole (307) extending in said head (300) from a second surface zone of said head (300) and joining said first hole (306), a stem (309) extending along said second hole (307) and formed with an aperture (310) therethrough aligned with said first hole (306), and a member (311) extending in said first hole (306) and said aperture (310) and retaining said head (300) on said stem (309), characterized in that said first hole (306) is closed at its end remote from said first surface zone and is closed at said first surface zone by a plug (312) therein.

2. A component according to claim 1, characterized in that said second hole (307) is of closed cross-section.

3. A component according to claim 1 or 2, characterized in that said first hole (306) is a blind bore.

## Revendications

1. Un élément de prothèse comprenant une tête articulaire (300), un premier trou (306) de section transversale fermée disposé dans la tête à partir d'une première zone de surface de cette tête (300) un second trou (307) disposé dans la tête (300) à partir d'une seconde zone de surface de la tête (300) et rejoignant le premier trou (306), une tige (309) disposée le long du second trou (307) et formée avec une ouverture (310) étant ainsi alignée avec le premier trou (306) et un élément (311) disposé dans le premier trou (306) et l'ouverture (310) et retenant la tête (300) sur la tige (309), caractérisé en ce que le premier trou (306) est fermé à son extrémité éloignée de la première zone de surface et est fermé à cette première zone de surface par un bouchon (312).

2. Un élément conforme à la revendication 1, caractérisé en ce que le second trou (307) est de section transversale fermée.

3. Un élément conforme à la revendication 1 ou 2, caractérisé en ce que le premier trou (206) est un alésage borgne.

**Patentansprüche**

1. Ein prothetisches Teil mit einem Gelenkkopf (300), einem sich von einem ersten Oberflächenbereich des Kopfes (300) in den Kopf erstreckenden, ersten Loch (306) mit geschlossenem Querschnitt, einem sich von einem zweiten Oberflächenbereich des Kopfes (300) in den Kopf erstreckenden und mit dem ersten Loch (306) zusammentreffenden, zweiten Loch (307), einem sich entlang des zweiten Loches (307) erstreckenden Schaft (309) mit einer auf das erste Loch (306) ausgerichteten Öffnung (310), und einem sich in das erste Loch (306) und die Öffnung (310) erstreckenden und den Kopf (300) auf dem Schaft (309) haltenden Glied (311), dadurch gekennzeichnet, daß das erste Loch (306) an dem von dem ersten Oberflächenbereich entfernten Ende geschlossen ist und an dem ersten Oberflächenbereich von einem Stopfen (312) verschlossen ist.

2. Ein Teil nach Anspruch 1, dadurch gekennzeichnet, daß das zweite Loch (307) einen geschlossenen Querschnitt aufweist.

3. Ein Teil nach Anspruch 1 oder 2, dadurch gekennzeichnet, daß es sich bei dem ersten Loch (306) um ein Sackloch handelt.

FIG. 1.

FIG. 2.

FIG. 3.

FIG. 4.

FIG. 5.

FIG. 6.

FIG. 7.

FIG. 8.

FIG. 9.

FIG. 10.

FIG. 11.

FIG. 12.

2

FIG. 13.

FIG. 14.

FIG. 16.

FIG. 15.

FIG. 17.

FIG. 18.

FIG. 20.

4

FIG. 19.

0 057 793